# EUROPEAN PATENT APPLICATION

(11) **EP 3 513 782 A1**
(43) Date of publication of application: **24.07.2019**
(21) Application number: 19151345.6
(22) Date of filing: 11.01.2019
(51) Int. Cl.: A61K 9/06, A61K 47/36

(54) **GEL FORMULATIONS FOR ORAL ADMINISTRATION OF DRUGS, IN PARTICULAR TO DYSPHAGIC PATIENTS**

(30) Priority: 18.01.2018 IT 201800001301
(71) Applicant: Universita' Degli Studi Di Camerino, 62032 Camerino (MC) (IT); Pharma & Food Consulting Srl, 62032 Camerino (MC) (IT); I.N.R.C.A. Istituto Nazionale Riposo E Cura Anziani, 60124 Ancona (AN) (IT)
(72) Inventor: BONACUCINA, Giulia, 62032 CAMERINO (MC) (IT); CESPI, Marco, 62032 CAMERINO (MC) (IT); CASETTARI, Luca, 61029 URBINO (PU) (IT); TORREGIANI, Elisabetta, 62032 CAMERINO (MC) (IT); LOGRIPPO, Serena, 62032 CAMERINO (MC) (IT); PALMIERI, Giovanni Filippo, 62032 CAMERINO (MC) (IT); PERINELLI, Diego Romano, 62032 CAMERINO (MC) (IT); GANZETTI, Roberta, 60035 JESI (AN) (IT); SESTILI, Matteo, 60037 MONTE SAN VITO (AN) (IT)
(74) Representative: Currado, Luisa

(57) **Abstract**

A pharmaceutical gel formulation is disclosed containing watersoluble therapeutically active ingredient which are generally available only as oral solid dosage forms or tablets and/or capsules and/or immediate release powders or granules, which can be administered in the presence of abnormalities in the swallowing process.

## Description

### Background of the invention

The present invention concerns the pharmaceutical field since it provides a gel formulation containing water-soluble active ingredients predominantly available in the pharmaceutical market as oral solid dosage forms such as instant-release tablets and capsules or powders or granules for oral use. The formulation object of the present invention satisfies the particular needs of patients showing problems in the swallowing process. The condition that generally defines the problems related to the swallowing act is called dysphagia.

### State of the art

Patients with swallowing difficulties, or dysphagia, are not able to take oral medications in solid form, such as tablets or capsules. (Stegemann, S., M. Gosch, and J. Breitkreutz. "Swallowing dysfunction and dysphagia is an unrecognized challenge for oral drug therapy." International journal of pharmaceutics 430.1 (2012): 197-206).

Swallowing is a complex function that involves different muscles of the mouth, throat and esophagus controlled by different cortical areas of the brain and by the center of swallowing located in the brainstem. The brain communicates with the muscles through different cranial nerves. Swallowing requires high coordination with breathing, and the lack of synchronized movements causes in the patient suffocation or possible pulmonary aspiration of the food bolus. The difficulties that the patient can meet to feed remain even when he has to take drug therapy.

The most common commercially available pharmaceutical forms are solid oral forms, such as capsules or tablets, but they can hardly be administered to all patients with difficulty swallowing, such as children (Batchelor, Hannah K., and John F. Marriott. "Formulations for children: problems and solutions. "British journal of clinical pharmacology 79.3 (2015): 405-418), elderly (Miller, N., and J. Patterson." Dysphagia: implications for older people. "Reviews in Clinical Gerontology 24.01 (2014): 41-57, Forster, A., et al. "Oropharyngeal dysphagia in older adults: a review." European Geriatric Medicine 2.6 (2011): 356-362), or patients bedridden and / or intubated (Salmon, Damien, et al. "Pharmaceutical and safety considerations of tablet crushing in patients undergoing enteral intubation." International journal of pharmaceutics 443.1 (2013): 146-153).

When only solid pharmaceutical forms are available on the market for a specific active ingredient, healthcare professionals must address and solve problems related to the manipulation and administration of drugs in patients with difficulty swallowing, especially if they are pediatric or geriatric (Fusco, S ., et al., "Management of oral drug therapy in elderly patients with dysphagia." Official Journal of the Italian Society of Gerontology and Geriatrics: 9). The problem of the administration of drugs in people suffering from dysphagia is particularly evident in the hospital, although it should not be underestimated also at home and in residential health care facilities (Kelly, Jennifer, Gibson D'Cruz, and David Wright "A qualitative study of the problems surrounding the administration of patients with dysphagia." Dysphagia 24.1 (2009): 49).

In everyday clinical practice, the administration of drug therapy in patients with swallowing deficiency, when adequate forms or routes of administration are not available, health care professionals are forced to manipulate the available tablets and / or capsules, by crushing or opening them, to reduce them in powder and then mix them with food, drink or gelled water, so as to obtain extemporaneous preparations to be administered to patients. This practice is neither risk-free nor for patients (Kelly, Jennifer, Gibson D'Cruz, and David Wright. "A qualitative study of the problems surrounding the administration of patients with dysphagia." Dysphagia 24.1 (2009): 49) nor for health care professionals. In fact, the risk of administering an improper dosage of the drug may incur, making it either toxic or ineffective. Furthermore, the grinding of the tablets and / or the opening of the capsules can alter the efficacy of the active ingredient, make it unstable, as in the case of pH-sensitive or photo-sensitive active ingredients, reducing its palatability where the active ingredients have a taste particularly bitter, and cause the partial loss, if the handling operations are not carried out with the utmost appropriateness (Logrippo, Serena, et al. "Oral drug therapy in elderly with dysphagia: between a rock and a hard place!" Interventions in Aging 12 (2017): 241-251).

Furthermore, the powders released during handling of the tablet or capsule can be inhaled by the health worker causing damage. Inhalation or more generally contact with powders containing active molecules may cause problems to the skin or mucous membranes. To prevent or limit the toxicity of the active molecules it should be necessary to work in dedicated environments and take all the necessary precautions. Cross contamination could also be an issue in the working environment and also in the instruments, such as tablet-cutter or mortars, which are used for the handling operations. Furthermore, if the instruments are not perfectly clean, they can contaminate subsequent extemporaneous preparations with traces of other previously manipulated powders.

The swallowing of the oral solid forms generally occurs by swallowing the tablet or capsule with a certain volume of water. Water or more generally liquids, however, are difficult to swallow for those who show functional changes in the act of swallowing. This will therefore be facilitated by taking foods or beverages with a modified consistency (Manrique-Torres, Yady Juliana, et al.) Crushed tablets: "Journal of Pharmacy & Pharmaceuticals". Sciences 17.2 (2014): 207-219). Consequently, in the hospital practice, the powder obtained from capsules or tablets or the granulate itself, are dispersed in semi-solid preparations and / or gels with a proper consistency to facilitate swallowing.

For example, the gelled water Resource® (Nestle Health Science, Vevey, Switzerland), a "flavored gelatinous beverage suitable for people who have difficulty swallowing fluids", is commercially available.

U.S. Patent Application No. US20080160087 discloses an oral gel preparation comprising an HMG-CoA reductase inhibitor or a pharmaceutically acceptable salt thereof, a gelling agent, a polymer, a buffering agent, a preservative agent, a sweetening agent, and water, and having pH between 7 and 10. The gelling agent can be agar, pectin, sodium alginate, carrageenan, carob gum, xanthan gum and guar gum, k-carrageenan, ι-carrageenan, or xanthan gum, and also combinations of two or more gelling agents selected from agar, non-ionic polysaccharides, polysaccharides having a sulfuric acid group and / or a carboxylic group, aminopolisaccharides, cellulose derivatives and proteins. The combinations listed are two or more substances selected from gelatin, milk casein, mannans, carrageenan, starch, gelatinised starch, tragacanth, tamarind seed polysaccharide, gellan, karaya gum, cassia gum, tara gum, chitosan, psyllium seeds, ghatti gums and similar, such as a combination of agar, ι-carrageenan, xanthan gum and carob seed flour, a combination of k-carrageenan, locust bean flour, guar gum and pectin, a combination of ι-carrageenan, k-carrageenine, carob seed flour and guar gum, and a combination of sodium salt of alginic acid, pectin, xanthan gum and agar. The percentage of preferred gelling agent is from 0.05 to 15% by weight, more preferably from 0.2 to 10% by weight, even more preferably from 0.5 to 5% by weight with respect to the total amount of the whole preparation.

The buffering agent may be lactic acid, citric acid, tartaric acid, malic acid, and adipic acid and/or inorganic acids such as phosphoric acid, and metal ions such as potassium ion, sodium ion, calcium ion and magnesium ion and/or hydroxides of these metal ions in an amount without limitations and preferably not less than 0.001% by weight and not more than 15% by weight, with respect to the total amount of the preparation. The listed preservatives are isobutyl para-oxybenzoate, isopropyl para-oxybenzoate, ethyl para-oxybenzoate, propyl para-oxybenzoate, methyl para-oxybenzoate, benzoic acid, sodium edetate, calcium disodium etetate, salicylic acid, sorbic acid, dihydroacetic acid and salts, chlorobutanol, benzyl alcohol, phenylethyl alcohol, phenoxyethanol, menthol, camphor, cetyl pyridinium chloride, benzethonium chloride, benzalkonium chloride and similar in an unlimited amount. The sweeteners used may be polyalcohols of glycerol, propylene glycol, D-sorbitol, xylitol, mannitol, erythritol, maltitol, trehalose, raffinose, sucrose, fructose, glucose and lactose in an unlimited quantity. The sweetener can be sodium saccharin, aspartame, stevia, glycyrrhizinic acid, isomaltulose and others. Preparatory examples of gels are reported where the preferred gelling agent is combinations of pectin, carob seed flour, xanthan gum, carrageenin k, agar in powder, microcrystalline cellulose, of which data concerning the maintenance of the gel, stability of preservation and palatability.

### Closest prior art

The international patent application, publication number WO2009/098520 discloses an edible gel composition for promoting the swallowing of solid pharmaceutical forms, which includes gelatin and HPMC, wherein gelatin is used as a gelling agent and hydroxypropylmethylcellulose plays the role of lubricating agent.

The above mentioned composition when administered is in solid gel form coating solid oral pharmaceutical forms such as tablets or capsules or the same are inserted into a cut or recess in the gel itself. This gel is a strong and elastic gel which has the characteristic to be a solid type (solid-like) pharmaceutical form like a gummy candy.

The US patent no. US 6277395 disclosed a gelled beverage in which solid pharmaceutical forms are crushed and dispersed in the already gelled medium to allow them to be swallowed.

### Technical problem

The present invention provides a method for reformulating therapeutically active, water-soluble drugs, when commercially available for oral administration only as immediate release solid dosage form, mostly tablets and/or capsules without overlooking the existence of granules and powders for use oral.

The possible availability of liquid forms for oral administration (for example syrups, oral solutions, granules to be dispersed in a suitable solvent) or parenteral (for example vials whose content could be thickened after adjustment of dosage and osmolarity of the liquid product), could be considered as a further possibility of reformulation of a commercial product adapting to the needs of the dysphagic patient.

The therapeutically active molecules are reformulated as semi-solid preparations suitable for oral administration in patients with difficulty in swallowing or dysphagia. The mentioned method allows obtaining a kinetics of release of the active principle unaltered, with respect to that of the active ingredient formulated in the original solid dosage form.

In the light of what is already known in the art, the inventors of the present invention have developed an alternative and more effective solution to the problem, capable of overcoming the difficulties discussed above.

In particular, with respect to the prior art closest to the invention, the present invention differs from the international patent application, publication number WO2009/098520, since it allows dispersion of an immediate release tablet or capsule containing a water-soluble active principle in a semi-solid formulation with rheological characteristics comparable to commercial gelled drinks, used for hydration and administration of therapy to patients with swallowing disorders, while the prior art document discloses a gel, which coats solid oral pharmaceutical forms, wherein is said gel the gelatin is used as a gelling agent, while the hydroxypropylmethylcellulose (HPMC) acts as lubricant and they are present in the formulation at the concentration of 2% and 0.4% by weight respectively. On the contrary, in the present invention, hydroxypropylmethylcellulose, dispersed in water, preferably at a concentration of 3.5% by weight, acts exclusively as a gelling agent.

The nature of the composition is reflected on the physical characteristics of the gel itself. The gel described in WO2009/098520 is not a weak gel that must flow under the action of gravity, being pourable, but on the contrary it is a strong and elastic gel that should not flow under gravity in order to constitute a solid-like solid form like gummy candies of determinate shapes and sizes. WO2009/098520 differs in the preparation of the gel itself, that consists in the preparation of a high temperature liquid "pre-gel" which is transformed into a solid cylinder through an injection molding or pouring process inside molds, and subsequent cooling; where the solid pharmaceutical form is subsequently inserted manually or mechanically into the cylinder by application of a force. The final product contains the solid pharmaceutical form as such.

The present invention also distinguishes from US patent no. US6277395, which describes a gelled beverage similar to products already present on the market, in which the tablets are crushed and dispersed in the already gelled medium.

The inventors of the present invention have made a proactive selection among the different possible polymers with thickening capacities known in the art, in order to identify those capable of guaranteeing a convenient administration of the active molecules present in the immediate release solid forms available on the market and at, the same time, not altering the release kinetics of the active ingredients themselves.

The preparation method allows to disperse the pharmaceutical forms available on the market as tablets, coated and uncoated, or the contents of capsules or immediate-release bags, directly into the solvent which will then be used for the preparation of the polymer dispersion, avoiding any manipulation operation and eliminating the dangers related to loss of active principle, cross contamination and dosing errors. In this way, the drug, which was originally available only in solid or liquid form not suitable for administration in dysphagic patients, is in a new dosage form of adequate consistency to swallowing, even in the dysphagic subject, without any alteration of its release kinetics, remaining as effective as in its original dosage form.

### Object of the invention

With reference to the attached claims, the technical problem is therefore solved by providing a composition comprising a semi-solid preparation consisting of:
hydroxypropylmethylcellulose in a percentage ranging from 1 to 6% by weight with respect to the total composition,
a pharmacologically effective amount of at least one water-soluble therapeutically active ingredient,
suitable pharmaceutically acceptable excipients
wherein the water-soluble therapeutically active ingredient is in the form of tablet and/or immediate release capsule for oral use, dispersed in the semisolid preparation.

The invention further includes a kit for preparing a semisolid composition for oral administration to dysphagic patients consisting of
a composition comprising a semi-solid preparation consisting of hydroxypropylmethylcellulose in a percentage comprised between 1 and 6% by weight with respect to the total composition,
suitable pharmaceutically acceptable excipients
at least one therapeutically active ingredient in the form of a tablet and/or immediate release capsule for oral use, to be dispersed in the semisolid preparation.

The invention also comprises the method of preparation of the semisolid preparation, providing that hydroxypropylmethylcellulose is dispersed under stirring at a temperature comprised between 60°C and 70°C in water as a solvent until complete solvation of the hydroxypropylmethylcellulose and subsequent cooling.

The invention also includes the method of preparation of the composition which provides for the following steps:
a) dispersion under stirring of the hydroxypropylmethylcellulose at a temperature between 60°C and 70°C in water as solvent until complete solvation of hydroxypropylmethylcellulose;
b) gradual cooling of the product obtained in step a) and addition of a pharmacologically effective amount of at least one water-soluble therapeutically active ingredient and suitable pharmaceutically acceptable excipients under stirring;
c) gelling of the product obtained in step b) at a temperature below 30°C until the final composition is obtained.

Another object of the present invention is a dosage regimen in which a composition comprising a semi-solid preparation consisting of hydroxypropylmethylcellulose in a percentage ranging from 1 to 6% by weight with respect to the total composition, a pharmacologically effective amount of at least one water-soluble therapeutically active ingredient and suitable pharmaceutically acceptable excipients,
wherein the water-soluble therapeutically active ingredient is in the form of capsule and/or a immediate release tablet for oral use, dispersed in the semisolid preparation, characterized in that said composition is administered orally to patients with difficulty in swallowing or dysphagia.

Furthermore, the invention also provides the composition comprising a semi-solid preparation consisting of hydroxypropylmethylcellulose in a percentage ranging from 1 to 6% by weight with respect to the total composition, a pharmacologically effective amount of pravastatin sodium salt and suitable pharmaceutically acceptable excipients, wherein the composition has a pH comprised between 6 and 10, and wherein the sodium salt of pravastatin is in the form of an immediate-release tablet for oral use, dispersed in the semisolid preparation, and the relative kit, for use in a method for the treatment of hypercholesterolemia .

As well as constituting a composition comprising a semi-solid preparation consisting of hydroxypropylmethylcellulose in a percentage ranging from 1 to 6% by weight with respect to the total composition, by a pharmacologically effective amount of pravastatin and by suitable pharmaceutically acceptable excipients, wherein the composition has a pH between 6 and 10 and wherein pravastatin is in the form of a tablet and/or a immediate release capsule for oral use, dispersed in the semisolid preparation for the treatment of hypercholesterolemia characterized in that said composition is administered orally to patients with difficulty in swallowing or dysphagia in a single daily dose.

### Brief description of figures

Figure 1 shows the results of rheological analysis by means of oscillation stress sweep tests at 25°C performed on Carragenine samples of types k-, λ- and ι- with respect to the Resource® reference.
Figure 2 shows the comparison between the easiness of administration to the teaspoon, the dispersion time (TD) in water and the elastic modulus (G') of a gelled water mono-dose with the different thickening polymers.
Figure 3 shows the release kinetics of the sodium salt of pravastatin in commercial tablets compared to the semi-solid preparations based on HEC HHX and GG.
Figure 4 shows the release kinetics of the sodium salt of pravastatin in commercial tablets compared to the semi-solid preparations based on HPMC at different concentrations 3%-3.5%-4%.
Figure 5 shows the rheological stability results up to 60 days of the semi-solid preparation based on 3.5% HPMC by oscillation stress sweep tests (G') for samples stored either at room temperature or in the refrigerator.
Figure 6 shows the results of the dissolution tests at 0-14-30-60 days of samples stored in two conditions, i.e. at room temperature and in the refrigerator, the data are within the established confidence interval of 95%.

### Detailed description of the invention

### Definitions

Within the meaning of the present invention, for semisolid compound, or vehicle, or base, is meant a hydrogel consisting of hydroxypropylmethylcellulose, also called hypromellose in European Pharmacopoeia 9th edition (https://www.edqm.eu/en/european-pharmacopoeia-9th-edition), a semi-synthetic cellulose polymer, in a percentage between 1 and 6% by weight with respect to the total composition.

Within the meaning of the present invention, water-soluble therapeutically active ingredient means any pharmaceutically and therapeutically active ingredient having a solubility in water or in aqueous solution and/or in any aqueous medium, including hydrogels, in the "soluble-very soluble" range, as defined according to the Italian Official Pharmacopoeia XII edition (http://www.iss.it/farc/), in reference to a temperature between 15 ° C and 25 ° C, we mean the approximate volume in ml necessary to dissolve one gram of substance: very soluble less than 1, freely soluble from 1 to 10, soluble from 10 to 30, sparingly soluble from 30 to 100, slightly soluble from 100 to 1000, very slightly soluble from 1000 to 10000, practically insoluble more than 10000.

Within the meaning of the present invention, pharmacologically effective amount of water-soluble therapeutically active ingredient means the quantity of therapeutically active water-soluble ingredient present in the original solid form, such as commercially available tablets and/or capsules.

Within the meaning of the present invention the water-soluble therapeutically active ingredient can be selected in the group consisting of families and/or classes of compounds defined according to the anatomical therapeutic and chemical classification system (ATC) (https://www.whocc.no/): drugs intended for the gastrointestinal tract and metabolism (group A), drugs intended for blood and hematopoietic system (group B), drugs for the cardiovascular system (group C), drugs for the integumentary system and skin (group D), drugs for the genito-urinary system and sex hormone (group G), drugs for the endocrine system, excluding sex hormones and insulin (group H), anti-infective drugs for systemic use (group J), antineoplastic drugs and immunomodulators (group L), drugs for the muscular system - skeletal system and joints (group M), drugs for the nervous system (group N), antiparasitic drugs, insecticides and repellents (group P), drugs for the respiratory system (group R), drugs for the sense organs (group S), various drugs (group V).

The water-soluble therapeutic ingredient can be selected in the group consisting of:
sodium fluoride, benzydamine hydrochloride, ranitidine hydrochloride, nizatidine, scopolamine butylbromide, metoclopramide monochloride monohydrate, metoclopramide hydrochloride, ondansetron hydrochloride dihydrate, sodium picosulfate, lactulose, polyethylene glycol (PEG) 4000 (macrogol 4000), polyethylene glycol mixture (PEG) 4000 (macrogol 4000) and anhydrous sodium sulfate and sodium bicarbonate and sodium chloride and potassium chloride, a mixture of polyethylene glycol (PEG) 3350 (macrogol 3350) and anhydrous sodium sulfate and sodium bicarbonate and sodium chloride and potassium chloride, a mixture of polyethylene glycol (PEG) 3350 (macrogol 3350) and sodium bicarbonate and sodium chloride and potassium chloride, mixture of polyethylene glycol (PEG) 3350 (macrogol 3350) and sodium sulfate and sodium chloride and potassium chloride and ascorbic acid and sodium ascorbate, paromomycin sulfate, vancomycin hydrochloride, chromoglicic acid disodium salt, metformin hydrochloride, gliclazide, acarbose, ascorbic acid, nicotinamide, pyridoxine hydrochloride, pantetine, zinc sulfate, levocarnitine, cysteamine bitartrate (mercaptamine bitartrate), zinc acetate, miglustat, warfarin sodium, lysine acetylsalicylate, tranexamic acid, ferrous gluconate, cobamamide, mexiletine hydrochloride, propafenone hydrochloride, flecainide acetate, ethylphrine hydrochloride, isosorbide mononitrate, trimetazidine dihydrochloride, clonidine hydrochloride, potassium canrenoate, pentoxifylline, naphtyhydrofuryl acid oxalate, troxerutin, propranolol hydrochloride, timolol maleate, sotalol hydrochloride, nadolol, metoprolol tartrate, celiprolol hydrochloride, verapamil hydrochloride, diltiazem hydrochloride, captopril, lisinopril dihydrate, perindopril arginine, perindopril erbumin (tert-butylamine), perindopril tosylate, quinapril hydrochloride, pravastatin sodium, oxibutinin hydrochloride, desmopressin acetate hydrate, betamethasone disodium phosphate, thiamazole (methimazole), thiamazole (methimazole)/dibromotyrosine, doxycycline hyclate, limecycline (tetracycline-levo-methylenlysine), tetracycline chloride rato, minocycline hydrochloride, bacampicillin hydrochloride, flucloxacillin sodium, clindamycin hydrochloride, lincomycin hydrochloride, ciprofloxaina hydrochloride monohydrate, ciprofloxaina hydrochloride, fosfomycin salt of trometamol, isoniazid, ethambutol hydrochloride, ribavirin, stavudine, telbivudine, inosine pranobex, cyclophosphamide, fludarabine phosphate, vinorelbine bitartrate, procarbazine hydrochloride, hydroxycarbamide, estramustine sodium phosphate, ketorolac, trometamol salt, naproxen sodium, glucosamine sulfate crystalline, cyclobenzaprine hydrochloride, colchicine, clodronic acid sodium salt, sodium ibandronate monohydrate, morphine sulphate, paracetamol/codeine phosphate, tramadol hydrochloride/paracetamol, oxycodone hydrochloride/paracetamol, tramadol hydrochloride, lysine acetylsalicylate, lysine acetylsalicylate/metoclopramide, monohydrochloride, paracetamol, paracetamol/ascorbic acid, paracetamol/pseudoephedrine hydrochloride, paracetamol/ascorbic acid/phenylephrine hydrochloride, paracet wharf/chlorfenamine maleate/sodium ascorbate, paracetamol/chlorfenamine maleate, paracetamol/pseudoephedrine hydrochloride/diphenhydramine hydrochloride, phenytoin sodium, sodium valproate, buxamine, vigabatrin, levetiracetam, methylhexyl hydrochloride monohydrate, amantadine hydrochloride, selegiline hydrochloride, chlorpromazine hydrochloride, trifluoperazine dihydrochloride, sugarylophexol dihydrochloride, clorazepate dipotassium, hydroxyzine dihydrochloride, flurazepam monohydrochloride, clomipramine hydrochloride, amitriptyline hydrochloride, dosulepin hydrochloride, oxitriptan, levoacetilcarnitina hydrochloride, amitriptyline hydrochloride/chlordiazepoxide hydrochloride, choline alfoscerate, naltrexone hydrochloride, lofexidine hydrochloride, betahistine dihydrochloride, tetrabenazine, chloroquine diphosphate, hydroxychloroquine sulfate , salbutamol sulfate, clenbuterol hydrochloride, bamifylline hydrochloride, doxofillin, acetylcysteine, diphenhydramine hydrochloride, chlorfenamine maleate, promethazine hydrochloride, deferiprone, calcium folinate.

The present invention provides a composition comprising
a semi-solid preparation consisting of hydroxypropylmethylcellulose in a percentage ranging from 1 to 6% by weight with respect to the total composition
a pharmacologically effective amount of at least one water-soluble water-soluble therapeutically active ingredient
and suitable pharmaceutically acceptable excipients
wherein the water-soluble water-soluble therapeutically active ingredient is in the form of a tablet and/or immediate release capsule for oral use, dispersed in the semisolid preparation.

The invention can be presented in the form of a kit containing the necessary to make a pharmacologically effective amount of at least one water-soluble water-soluble therapeutically active ingredient in the form of a tablet and/or immediate release capsule for oral use orally administered to patients with difficulty swallowing or dysphagia. Therefore, for this purpose, the kit consists of a composition comprising a semi-solid preparation consisting of hydroxypropylmethylcellulose in a percentage ranging from 1 to 6% by weight with respect to the total composition, suitable pharmaceutically acceptable excipients.

Another object of the present invention is a dosage regimen wherein a composition comprising a semi-solid preparation consisting of hydroxypropylmethylcellulose in a percentage ranging from 1 to 6% by weight with respect to the total composition, a pharmacologically effective amount of at least one water-soluble therapeutically active ingredient and suitable pharmaceutically acceptable excipients, wherein the water-soluble therapeutically active ingredient is in the form of a tablet and/or immediate release capsule for oral use, dispersed in the semisolid preparation, and characterized in that said composition is administered orally to patients with difficulty swallowing or dysphagia.

Preferably, the hydroxypropylmethylcellulose is in a percentage of 3.5% by weight with respect to the total composition.

The pharmaceutically acceptable excipients are sweetening and flavoring agents that make the product not only palatable but also suitable for pediatric and diabetic patients, while the preservative agents guarantee the microbiological stability of the preparation.

The sweetening agents are selected from the group comprising fructose, sorbitol, mannitol, xylitol, sucrose.

Preferably the sweetening agent is sorbitol.

The sweetening agents are preferably present in a concentration between 5% and 15% by weight with respect to the total composition, and even more preferably the concentration is 10%, with respect to the total composition.

The flavoring agents serve to mask the bitter taste of the active compound and are selected from the group comprising liquid cherry flavor, mandarin-vanilla flavoring in powder form, banana powder flavor, vanilla flavoring in powder form and mixtures and mixtures thereof.

Preferably the flavoring agent is mandarin-vanilla flavoring in powder form.

The flavoring agents are preferably present in a concentration ranging from 0.10% to 0.30% by weight with respect to the total composition, and even more preferably the concentration is 0.20%, with respect to the total composition.

The preservative agents are selected from the group consisting of derivatives of para-hydroxybenzoic acid, its salts and esters and mixtures thereof.

Preferably, the para-hydroxybenzoic acid derivatives are selected from the group consisting of sodium methyl parahydroxybenzoate, sodium propyl parahydroxybenzoate and mixtures thereof.

Preferably, the preservative agents are a mixture of sodium methyl parahydroxybenzoate and sodium propyl parahydroxybenzoate. Preferably, the preservative agents are present in a concentration between 0.10% and 0.25%, with respect to the total composition.

The water-soluble therapeutically active ingredient has a solubility within the "soluble-very soluble" range as defined by the Italian Official Pharmacopoeia XII edition (http://www.iss.it/farc/), i.e. with reference to a temperature between 15°C and 25°C, as the approximate volume in ml necessary to dissolve one gram of substance: very soluble less than 1, freely soluble from 1 to 10, soluble from 10 to 30.

Preferably, the water-soluble therapeutically active ingredient has a solubility of at least 10-30 ml of solvent for a part by weight of solute.

Preferably, the therapeutically active ingredient is selected from the group consisting of drugs intended for the gastrointestinal tract and metabolism (group A), drugs intended for blood and hematopoietic system (group B), drugs for the cardiovascular system (group C), drugs intended for tegumentary and skin (group D), drugs for the genito-urinary system and sex hormone (group G), drugs for the endocrine system, excluding sex hormones and insulin (group H), anti-infective agents for systemic use (group J), antineoplastic drugs and immunomodulators (group L), drugs for the muscular system - skeletal system and joints (group M), drugs for the nervous system (group N), antiparasitic drugs, insecticides and repellents (group P), drugs intended for respiratory system (group R), drugs intended for sense organs (group S), various drugs (group V) and mixture of them.

More preferably, the water-soluble therapeutically active ingredient is selected from the group consisting of:
sodium fluoride, benzydamine hydrochloride, ranitidine hydrochloride, nizatidine, scopolamine butylbromide, metoclopramide monochloride monohydrate, metoclopramide hydrochloride, ondansetron hydrochloride dihydrate, sodium picosulfate, lactulose, polyethylene glycol (PEG) 4000 (macrogol 4000), polyethylene glycol mixture (PEG) 4000 (macrogol 4000) and anhydrous sodium sulfate and sodium bicarbonate and sodium chloride and potassium chloride, a mixture of polyethylene glycol (PEG) 3350 (macrogol 3350) and anhydrous sodium sulfate and sodium bicarbonate and sodium chloride and potassium chloride, a mixture of polyethylene glycol (PEG) 3350 (macrogol 3350) and sodium bicarbonate and sodium chloride and potassium chloride, mixture of polyethylene glycol (PEG) 3350 (macrogol 3350) and sodium sulfate and sodium chloride and potassium chloride and ascorbic acid and sodium ascorbate, paromomycin sulfate, vancomycin hydrochloride, chromoglicic acid disodium salt, metformin hydrochloride, gliclazide, acarbose, ascorbic acid, nicotinamide, pyridoxine hydrochloride, pantetine, zinc sulfate, levocarnitine, cysteamine bitartrate (mercaptamine bitartrate), zinc acetate, miglustat, warfarin sodium, lysine acetylsalicylate, tranexamic acid, ferrous gluconate, cobamamide, mexiletine hydrochloride, propafenone hydrochloride, flecainide acetate, ethylphrine hydrochloride, isosorbide mononitrate, trimetazidine dihydrochloride, clonidine hydrochloride, potassium canrenoate, pentoxifylline, naphtyhydrofuryl acid oxalate, troxerutin, propranolol hydrochloride, timolol maleate, sotalol hydrochloride, nadolol, metoprolol tartrate, celiprolol hydrochloride, verapamil hydrochloride, diltiazem hydrochloride, captopril, lisinopril dihydrate, perindopril arginine, perindopril erbumin (tert-butylamine), perindopril tosylate, quinapril hydrochloride, pravastatin sodium, oxibutinin hydrochloride, desmopressin acetate hydrate, betamethasone disodium phosphate, thiamazole (methimazole), thiamazole (methimazole)/dibromotyrosine, doxycycline hyclate, limecycline (tetracycline-levo-methylenlysine), tetracycline chloride rato, minocycline hydrochloride, bacampicillin hydrochloride, flucloxacillin sodium, clindamycin hydrochloride, lincomycin hydrochloride, ciprofloxaina hydrochloride monohydrate, ciprofloxaina hydrochloride, fosfomycin salt of trometamol, isoniazid, ethambutol hydrochloride, ribavirin, stavudine, telbivudine, inosine pranobex, cyclophosphamide, fludarabine phosphate, vinorelbine bitartrate, procarbazine hydrochloride, hydroxycarbamide, estramustine sodium phosphate, ketorolac, trometamol salt, naproxen sodium, glucosamine sulfate crystalline, cyclobenzaprine hydrochloride, colchicine, clodronic acid sodium salt, sodium ibandronate monohydrate, morphine sulphate, paracetamol/codeine phosphate, tramadol hydrochloride/paracetamol, oxycodone hydrochloride/paracetamol, tramadol hydrochloride, lysine acetylsalicylate, lysine acetylsalicylate/metoclopramide, monohydrochloride, paracetamol, paracetamol/ascorbic acid, paracetamol/pseudoephedrine hydrochloride, paracetamol/ascorbic acid/phenylephrine hydrochloride, paracet wharf/chlorfenamine maleate/sodium ascorbate, paracetamol/chlorfenamine maleate, paracetamol/pseudoephedrine hydrochloride/diphenhydramine hydrochloride, phenytoin sodium, sodium valproate, buxamine, vigabatrin, levetiracetam, methylhexyl hydrochloride monohydrate, amantadine hydrochloride, selegiline hydrochloride, chlorpromazine hydrochloride, trifluoperazine dihydrochloride, sugarylophexol dihydrochloride, clorazepate dipotassium, hydroxyzine dihydrochloride, flurazepam monohydrochloride, clomipramine hydrochloride, amitriptyline hydrochloride, dosulepin hydrochloride, oxitriptan, levoacetilcarnitina hydrochloride, amitriptyline hydrochloride / chlordiazepoxide hydrochloride, choline alfoscerate, naltrexone hydrochloride, lofexidine hydrochloride, betahistine dihydrochloride, tetrabenazine, chloroquine diphosphate, hydroxychloroquine sulfate , salbutamol sulfate, clenbuterol hydrochloride, bamifylline hydrochloride, doxofillin, acetylcysteine, diphenhydramine hydrochloride, chlorfenamine maleate, promethazine hydrochloride, deferiprone, calcium folinate.

Preferably, the water-soluble therapeutically active ingredient is at least one inhibitor of the enzyme hydroxymethylglutaryl-CoA reductase, which belongs to the group of drugs intended for the cardiovascular apparatus (group C).

More preferably, the water-soluble therapeutically active ingredient is the sodium salt of pravastatin.

The concentration of the water-soluble therapeutically active ingredient is analogous to the concentration originally present in the tablet from which it derives.

The invention also includes the method of preparing the semi-solid preparation, which provides that the polymer is dispersed under stirring at a temperature of between 60°C and 70°C in water as solvent until the complete solvation of the polymer and subsequently cooling.

The invention also includes the method of preparation of the composition, which provides for the following steps
a) dispersion under stirring of the polymer at a temperature of between 60°C and 70°C in water until complete dispersion of the polymer;
b) gradual cooling of the product obtained in step a) and addition under stirring of a pharmacologically effective amount of at least one water-soluble therapeutically active ingredient in the form of immediate-release tablet for oral use and suitable pharmaceutically acceptable excipients;
c) gelling of the product obtained in step b) at a temperature below 30°C until the final composition is obtained.

Preferably the polymer is dispersed at a temperature of 65°C.

The stirring can be mechanical, for example by the use of blades or magnetic, for example by magnetic stirrers.

In a preferred embodiment of the present invention, the composition object of the present invention is constituted by hydroxypropylmethylcellulose (HPMC) 3.5%, sodium salt of pravastatin, sorbitol as sweetening agent, mandarin and vanilla powder as flavoring agents, and parabens as preservatives.

In a further preferred embodiment, the composition object of the present invention consists of:
Hydroxypropylmethylcellulose (HPMC) 3.5%
Sorbitol (10%)
Mandarin-vanilla powder flavor (0.20%)
Sodium methyl parahydrobenzoate (0.15%)
Sodium propyl parahydroxybenzoate (0.10%)
At least one tablet of sodium salt of pravastatin
Water qba 100gr
The product can be packaged in mono-doses of 5 gr.

Furthermore, the invention also provides the composition comprising a semi-solid preparation consisting of hydroxypropylmethylcellulose in a percentage ranging from 1 to 6% by weight with respect to the total composition, a pharmacologically effective amount of pravastatin sodium salt and suitable pharmaceutically acceptable excipients, wherein the composition has a pH between 7 and 10, and in which the sodium salt of pravastatin is in the form of immediate-release tablet for oral use, dispersed in the semisolid preparation, and the relative kit, for use in a method for the treatment of hypercholesterolemia.

As well as constituting a composition comprising a semi-solid preparation consisting of hydroxypropylmethylcellulose in a percentage ranging from 1 to 6% by weight with respect to the total composition, a pharmacologically effective amount of pravastatin sodium salt and suitable pharmaceutically acceptable excipients wherein the composition has a pH including between 7 and 10 in which the water-soluble therapeutic principle is in the form of an immediate-release tablets for oral use, dispersed in the semisolid preparation, for use in a method for the treatment of hypercholesterolemia characterized in that such a composition is administered orally to patients with difficulty swallowing or dysphagia.

Preferably, when the active substance is sodium salt of pravastatin for use in a method for the treatment of hypercholesterolemia, the oral administration is in a single daily dose.

### Examples

A hydrogel was developed comprising sodium salt of pravastatin having a release kinetics comparable to that of the solid form (tablet) for oral administration and commercially available.

To this end, a careful evaluation of several thickening polymers was carried out, on the base of their rheological properties (preferably the elastic modulus G'), their dispersion time (TD) in water and their easiness of administration (or administrability) by a teaspoon.

The following polymers were evaluated: xanthan gum (XG), guar gum (GG), carrageenans (C) of different types k-, λ- and ι- (in particular the types κ-911, κ-812, λ- 109, λ-209, ι-TPH-1), hydroxyethyl cellulose (HEC, HHX types, G and M), carboxymethylcellulose (CMC), carob seed flour (LBG), hydroxypropylmethylcellulose (HPMC), guar gum mixture and flour of carob seed (GG / LBG), gelled water prepared in the laboratory (LMGW).

All gelled vehicles were prepared by dispersing the polymers in water at a concentration of 1.25%. This percentage was initially arbitrarily established to evaluate the rheological response of each polymer. Subsequently, the percentage was adjusted on the base of the polymer examined, the rheological results and the visual evaluation of the obtained systems.

The gels were prepared at room temperature for all the polymers, except those of hydroxypropylmethylcellulose, which required a temperature of about 65 ° C and subsequent cooling at room temperature.

Then the gels obtained were evaluated by: a) easiness of administration by means of a teaspoon, b) rheological properties and in particular the solid component of the system or the elastic modulus (G '), c) time of dispersion in water (TD) to mimic the conditions in which the tablet is evaluated in terms of disintegration time in an aqueous vehicle.

The easiness of administration of the gel was evaluated with a test (panel test) using a teaspoon. The evaluation was performed by 25 anonymous examiners, who gave an opinion based on a preestablished score: score 0-3 for systems not suitable for use with a teaspoon, score 4-6 for products difficult to administer by teaspoon, and score 7-10 for simple administration products using a teaspoon.

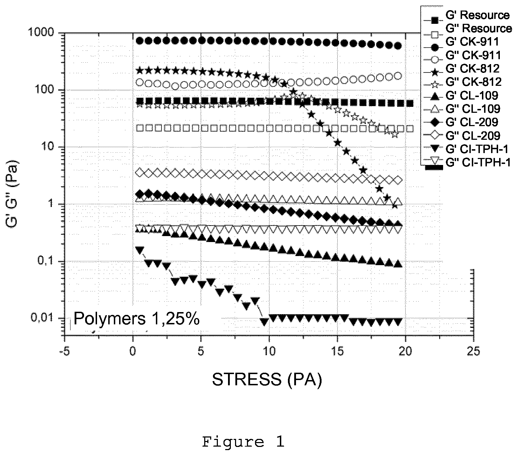

The rheological tests were performed at 25 ° C by means of a rotational rheometer equipped with 40/4 cone-flat geometry. In particular, the structural characteristics of the gels (elastic modules G 'and viscous G' ') were evaluated by increasing the applied stresses (shear stress) from 0 to 20 Pascal. An example of the results of rheological analysis is shown in Figure 1.

The dispersion time (TD) of each gelled system (erosion) in an aqueous medium was measured to identify which polymers would guarantee complete dispersion (erosion) of the gel in a time sufficiently comparable to that of an uncoated simple tablet. According to the Italian Pharmacopoeia XII edition, section 2.9.1, "disaggregation time of tablets and capsules" (http://www.iss.it/farc/), the disaggregation time for an uncoated simple tablet is within 15 minutes.

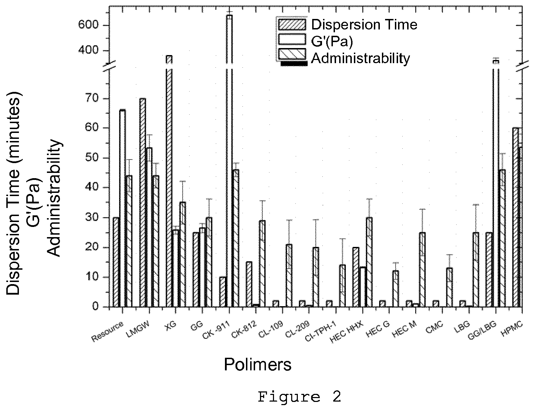

The results obtained from this first evaluation, and shown in figure 2, allowed to identify guar gum, κ-carrageenin 911 NF, κ-carrageenin 812 NF, HEC 250 HHX and HPMC as the most promising polymers for subsequent development. The pH values were checked in the 5 selected gelling polymers. A pH range of 7-10 is required both in the gelled vehicle and in the final formulation to ensure the chemical stability of the pravastatin sodium salt. In addition, pH levels of 11 or higher may cause adverse reactions in the gastrointestinal tract, such as stomatitis. On the contrary, more acidic values can however cause lesions of the oros-esophageal mucosa.

The aqueous dispersions obtained with four of the five selected polymers had pH values in the required range, as shown in Table 1. The system containing GG (water and guar gum) had a lower pH value, however, in the complete formulation obtained with the dispersed tablets, preserving agents, flavoring agent and sweetening agent, the final pH was within the required range. Therefore, these pH values obtained in the vehicles set up, made it possible to simply use water without having to resort to the use of basic solutions (for example, aqueous sodium bicarbonate solution of 8.4% or similar). These pH values simplify the formulation and in any case ensure a gelled vehicle suitable for the pravastatin sodium salt.

**Table 1**

| Polymers 1.25% | pH |
|---|---|
| Resource® | 7.00 |
| Cκ-911 | 7.72 |
| Cκ-812 | 7.61 |
| HEC HHX | 7.10 |
| GG* | 6.41 |

Then, the sweetening and flavoring agents were selected to formulate a gel with organoleptic characteristics suitable for oral administration.

Five sweeteners with different sweetening power such as saccharin, fructose, sorbitol, mannitol, xylitol were evaluated. Saccharin that has a sweetening power equal to 500 times that of sucrose, was discarded due to a possible incompatibility with the iron oxide, an excipient commonly found in tablets as in the commercial product used. Fructose, with sweetening power equal to 1.3-1.7 times that of sucrose, has been selected because it can be used by pediatric and diabetic patients. Sorbitol, with a sweetening power equal to 0.6 times sucrose, has been selected because it can be used by pediatric and diabetic patients. Mannitol with sweetening power of 0.5 and xylitol with sweetening power equal to 1 times that of sucrose were also selected.

The flavoring agents have been selected to mask the bitter taste of the active compound. The flavored flavoring agents were cherry flavoring in liquid form, while tangerine-vanilla flavoring, banana flavor, vanilla flavoring were in powder form.

Mixtures of sweetening agent / flavoring agent in water were prepared in different percentages to evaluate which composition had a better palatability in the presence of pravastatin. The sorbitol / mandarin-vanilla blend was selected as the most suitable after a panel test performed during the study. Sorbitol is used up to 33% for oral administration according to the US Food and Drug Administration (FDA).

The gel formulation was then optimized by varying the concentration of the selected polymers and evaluating the stability. The gels obtained with k-carrageenan 911 and sweetener/flavoring agent were discarded because they were subject to syneresis. The gels obtained with k-carrageenan 812 were discarded due to the impossibility of optimizing the formulation. In fact, this polymer has a low thickening power and also by varying its concentration, it is not possible to significantly vary the final consistency of the gel. Gels based on hydroxyethyl cellulose of the HHX type and guar gum (GG) showed adequate properties in terms of consistency and disintegration times at concentrations of 1.5% -1.75% and 1.5%, respectively. Gels prepared with HPMC were also suitable for administration in patients with dysphagia in the concentration range of 3-4%.

To define the final formulation, the release kinetics of the selected active ingredient (pravastatin sodium) were determined. The kinetics obtained were then compared with those of the dosage form on the market. All gels were prepared by adding to the base vehicle the active ingredient sodium salt of pravastatin as tablets dispersed in the gel.

Studies of kinetics of sodium salt release of pravastatin gel sodium were performed in compliance with the established parameters for sodium salt tablets of pravastatin by the US FDA: water as dissolution medium, volume of dissolution medium 900 ml, temperature of dissolution 37 ° ± 0.5 ° C, type II dissolution apparatus (paddle), paddle speed 50 revolutions per minute (rpm).

The apparatus used for the dissolution studies (Sotax) was coupled to a UV-Visible spectrophotometer (1800-Shimadzu) and the analyses were performed in triplicate, i.e. 3 mono-doses of the same gel analyzed in parallel. Every single dose of hydrogel was inserted into the vessel (vessel) of the dissolution apparatus through a common syringe where the final end had been previously cut, so that by pushing the plunger, the gel fell into the vessel without undergoing changes in structure and consistency. The amount of pravastatin released from the gel was analytically quantified at set times (0-5-15-30-60-90-120-180-240-300-360 minutes) through UV analysis at a fixed wavelength of 238nm (maximum peak of sodium salt absorption of pravastatin).

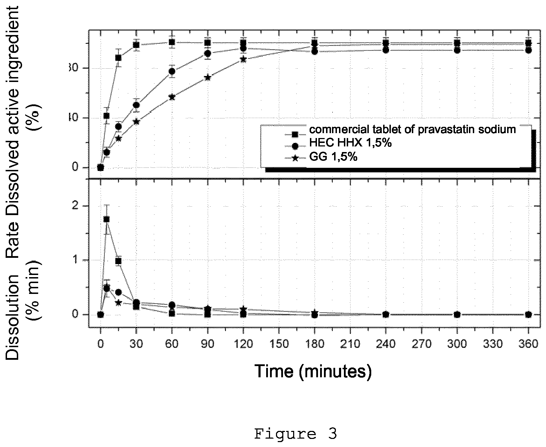

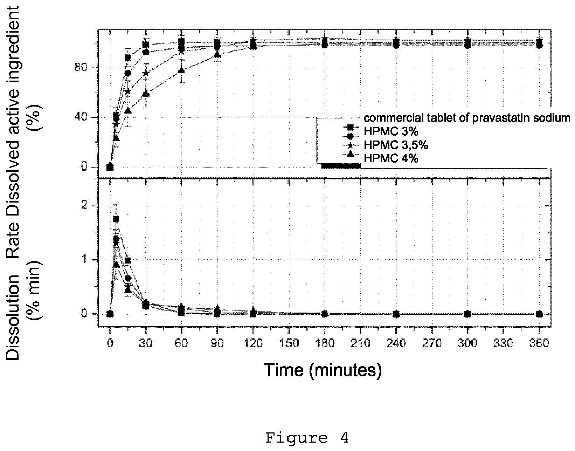

The results of the dissolution tests performed on gel loaded with pravastatin (shown in figure 3), allowed to discard the systems containing HEC and GG since the drug's release kinetics was excessively slow and did not correspond with an immediate release. In this regard, the best hydrogel contained HPMC at 3-3.5% concentration, as shown in Figure 4. However, taking into account the results of the rheological analysis and the results related to the administrability, the concentration of the 3.5% is to be preferred. This percentage guarantees the obtainment of a product that is easier to administer and swallow in dysphagic patients as well as ensuring a release of the active ingredient comparable to that of the reference tablet.

Finally, the studies performed allowed to select as the best formulation for the administration of drugs to dysphagic patients that consisting of 3.5% HPMC as a thickening agent, pravastatin sodium salt from tablets, parabens as preservatives, mandarin-vanilla powder as flavoring agent, sorbitol as a sweetening agent and water as a solvent. The purpose of the selection was to obtain a hydrogel that showed a release kinetic of the active ingredient from the gelled pharmaceutical form similar to that of a commercial tablet because the patient must receive the same dose at the same time also when the pharmaceutical dosage form administered is changed.

The substitution of pravastatin tablets with this new gel formulation makes drug therapy possible in patients who have developed inability to swallow during their lifetime.

Once the formulation was defined, the reproducibility of the batches and their stability were evaluated. Reproducibility was evaluated on three different batches in terms of rheological and release kinetic properties. The three batches showed stable and overlapping G 'elastic modules in the applied stress interval, while minimal dissimilarities were found in the release profiles of pravastatin from the dissolution test.

After defining the gel formulation, stability studies were conducted on the hydrogel containing 3.5% HPMC and sodium salt of pravastatin in the form of dispersed tablet. The stability of the active substance and formulation was assessed for up to 60 days under two different storage conditions, room temperature (22 ° -25 ° C) and refrigerator (2 ° -8 ° C), without exposure of the samples to light. Specifically, we evaluated both a variation of the mechanical properties of the gel (physical stability) as a function of the conservation condition and a possible reduction of the concentration of the active ingredient (chemical stability) of the product itself. Furthermore, the microbiological stability of the product was evaluated to verify its shelf life up to 60 days and therefore if the preservatives used were suitable both qualitatively and quantitatively. Throughout the period, pH measurements were also carried out.

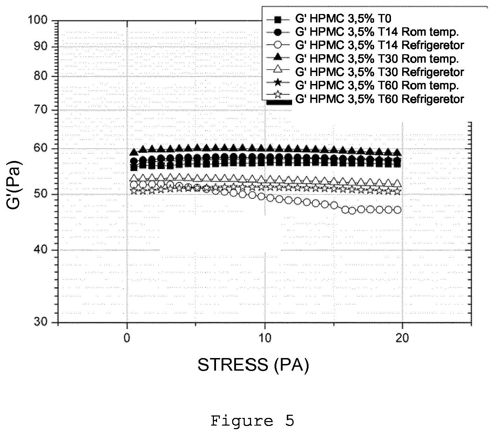

In particular, rheological investigations (physical stability) have shown that the elastic modulus G 'remains constant throughout the period considered (60 days), independently of the storage conditions, ensuring a gel structure suitable for administration in dysphagic patients, as shown in figure 5.

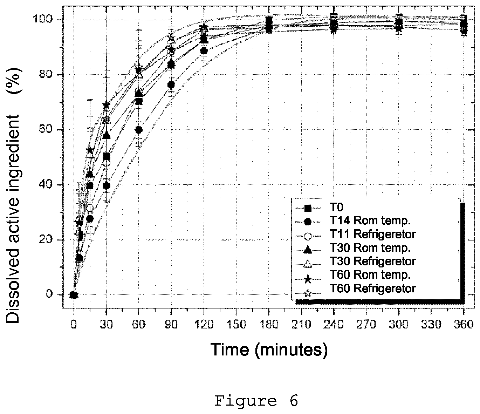

The stability studies of the kinetic profile allowed to monitor simultaneously any variations in the release and chemical stability kinetics of the active ingredient under examination. Analytical determinations were obtained by analyzing samples at 0-14-30-60 days stored at room temperature and in the refrigerator. All the curves obtained during the stability tests are within the 95% confidence interval defined during the reproducibility tests, as shown in Figure 6.

Regarding the microbiological tests, evaluations were made for the total aerobic microorganism count (CMAT) and for the total mold and yeast count (CFLT). The validation of the method and the materials used was performed by means of standard ATCC (American Type Culture Collection) strains. Specifically for plate sowing, Columbia Agar 5% sheep blood and Sabouraud gentamicin chloramphenicol for the CMAT and CFLT counts were used as culture media. According to the Italian Pharmacopoeia XII edition, section 5.14, "Microbiological quality of pharmaceutical preparations of substances for non-sterile pharmaceutical use" (http://www.iss.it/farc/), preparations for oral use must meet the following criteria: for CMAT 200 colony forming units (UFC) / mL as the maximum acceptable number and for CFLT 20 CFU / mL as the maximum acceptable number. The results obtained showed a lack of growth after the respective incubation periods and can be expressed as < 10 CFU / ml for both the plates used for the CMAT count and the CFLT count.

The pH measurements of the gelled product (3.5% HPMC plus dispersed tablets) during the 60 days of the stability study did not show any significant changes, as shown in Table 2.

**Table 2.**

| Days | pH at room temperature | pH in the refrigerator |
|---|---|---|
| 0 | 9.08 | |
| 14 | 9.01 | 9.05 |
| 30 | 8.86 | 9.05 |
| 60 | 8.61 | 8.91 |

## Claims

1. composition comprising
a semi-solid preparation consisting of hydroxypropylmethylcellulose in a percentage ranging from 1 to 6% by weight with respect to the total composition
a pharmacologically effective amount of at least one water-soluble therapeutically active ingredient
and suitable pharmaceutically acceptable excipients
wherein the water-soluble therapeutically active ingredient is in the form of a tablet and/or immediate release capsule for oral use, dispersed in the semisolid preparation.

2. Composition according to claim 1 wherein the water-soluble therapeutically active ingredient is selected from the group consisting of: drugs intended for the gastrointestinal tract and metabolism, drugs intended for the blood and hematopoietic system, drugs for the cardiovascular apparatus, drugs intended for the apparatus integumentary and skin, drugs intended for the genito-urinary apparatus and sex hormone, drugs intended for the endocrine system, excluding sex hormones and insulin, anti-infective drugs for systemic use, antineoplastic drugs and immunomodulators, drugs for the muscular system and skeletal system and joints, drugs for the nervous system, antiparasitic drugs, insecticides and repellents, drugs for the respiratory system, drugs for sensory organs, various drugs.

3. Composition according to claim 2 where the water-soluble therapeutically active ingredient is selected from the group consisting of: sodium fluoride, benzydamine hydrochloride, ranitidine hydrochloride, nizatidine, scopolamine butylbromide, metoclopramide monochloride monohydrate, metoclopramide hydrochloride, ondansetron hydrochloride dihydrate, sodium picosulfate, lactulose , polyethylene glycol (PEG) 4000 (macrogol 4000), mixture of polyethylene glycol (PEG) 4000 (macrogol 4000) and sodium sulfate anhydrous and sodium bicarbonate and sodium chloride and potassium chloride, mixture of polyethylene glycol (PEG) 3350 (macrogol 3350) and sodium sulfate anhydrous sodium bicarbonate and sodium chloride and potassium chloride, polyethylene glycol mixture (PEG) 3350 (macrogol 3350) and sodium bicarbonate and sodium chloride and potassium chloride, polyethylene glycol mixture (PEG) 3350 (macrogol 3350) and sodium sulfate and sodium chloride and potassium chloride and ascorbic acid and sodium ascorbate, paromomycin sulfate, vancomycin hydrochloride, acid cr homoglicic disodium salt, metformin hydrochloride, gliclazide, acarbose, ascorbic acid, nicotinamide, pyridoxine hydrochloride, pantetine, zinc sulphate, levocarnitine, cysteamine bitartrate (mercaptamine bitartrate), zinc acetate, miglustat, sodium warfarin, lysine acetylsalicylate, tranexamic acid, ferrous gluconate, cobamamide, mexiletine hydrochloride, propafenone hydrochloride, flecainide acetate, ethylphrine hydrochloride, isosorbide mononitrate, trimetazidine dihydrochloride, clonidine hydrochloride, potassium canrenoate, pentoxifylline, naphtyhydrofuryl acid oxalate, troxerutin, propranolol hydrochloride, timolol maleate, sotalol hydrochloride, nadolol, metoprolol tartrate, celiprolol hydrochloride , verapamil hydrochloride, diltiazem hydrochloride, captopril, lisinopril dihydrate, perindopril arginine, perindopril erbumin (tert-butylamine), perindopril tosylate, quinapril hydrochloride, pravastatin sodium, oxibutinin hydrochloride, desmopressin acetate hydrate, betamethasone disodium phosphate, tiamazole (m etimazole), tiamazole (methimazole)/dibromotyrosine, doxycycline hyclate, limecicline (tetracycline-levo-methylenlysine), tetracycline hydrochloride, minocycline dihydrochloride, bacampicillin hydrochloride, flucloxacillin sodium, clindamycin hydrochloride, lincomycin hydrochloride, ciprofloxaine hydrochloride monohydrate, ciprofloxaine hydrochloride, phosphomycin trometamol, isoniazid, ethambutamine hydrochloride, ribavirin, stavudine, telbivudine, metisoprinol, cyclophosphamide, fludarabine phosphate, vinorelbine bitartrate, procarbazine hydrochloride, hydroxycarbamide, estramustine sodium phosphate, ketorolac trometamol salt, naproxen sodium, glucosamine sulfate crystalline, cyclobenzaprine hydrochloride, colchicine, acid clodronic sodium salt, sodium ibandronate monohydrate, morphine sulfate, paracetamol/codeine phosphate, tramadol hydrochloride/paracetamol, oxycodone hydrochloride/paracetamol, tramadol hydrochloride, lysine acetylsalicylate, lysine acetylsalicylate/metoclopramide, monohydrochloride, paracet amol, paracetamol/ascorbic acid, paracetamol/pseudoephedrine hydrochloride, paracetamol/ascorbic acid/phenylphrine hydrochloride, paracetamol/chlorfenamine maleate/sodium ascorbate, paracetamol/chlorfenamine maleate, paracetamol/pseudoephedrine hydrochloride/diphenhydramine hydrochloride, phenytoin sodium, sodium valproate, buxamine, vigabatrin, levetiracetam, metixene hydrochloride monohydrate, amantadine hydrochloride, selegiline hydrochloride, chlorpromazine hydrochloride, trifluoperazine dihydrochloride, zuclopenthixol dihydrochloride, clorazepate dipotassium, hydroxyzine dihydrochloride, flurazepam monohydrochloride, clomipramine hydrochloride, amitriptyline hydrochloride, dosulepin hydrochloride, oxitriptan, levoacetilcarnitina hydrochloride, amitriptyline hydrochloride/chlordiazepoxide hydrochloride, choline alfoscerate, naltrexone hydrochloride, lofexidine hydrochloride, betahistine dihydrochloride, tetrabenazine, chloroquine diphosphate, hydroxychloroquine sulfate, salbutamol sulfate, clenbuterol hydrochloride, bamifylline hydrochloride, doxofillina, acetylcysteine, diphenhydramine hydrochloride, chlorfenamine maleate, promethazine hydrochloride, deferiprone, calcium folinate.

4. Composition according to claim 1 comprising
Hydroxypropylmethylcellulose 3.5%
Sorbitol 10%
Tangerine-vanilla powder aroma 0,20%
Sodium methyl parahydroxybenzoate 0.15%
Sodium propyl para-hydroxybenzoate 0,10%
At least one tablet of pravastatin sodium salt
Water qba 100g

5. Composition according to claim 4 for the treatment of hypercholesterolemia.

6. Kit for the preparation of a semisolid composition for oral administration to dysphagic patients consisting of a pharmacologically effective amount of at least one water-soluble therapeutically active ingredient, in the form of a tablet and/or capsule and/or powder or granules, for immediate release to be dispersed, a semi-solid preparation consisting of hydroxypropylmethylcellulose in a percentage ranging from 1 to 6% by weight with respect to the total composition, suitable pharmaceutically acceptable excipients.

7. Method for the preparation of a semi-solid preparation consisting of hydroxypropylmethylcellulose in a percentage ranging from 1 to 6% by weight with respect to the total composition of claim 1 wherein hydroxypropylmethylcellulose is dispersed in water under stirring at a temperature of between 60°C and 70°C until complete solvation of the same and subsequent cooling.

8. Method for preparing the composition of claim 1 comprising the following steps
a) dispersion under stirring of hydroxypropylmethylcellulose at a temperature of between 60°C and 70°C in water until complete dispersion of the same;
b) gradual cooling of the product obtained in step a) and addition of a pharmacologically effective amount of at least one water-soluble therapeutically active ingredient in the form of immediate-release tablet for oral use and suitable pharmaceutically acceptable excipients under stirring;
c) gelling of the product obtained in step b) at a temperature below 30°C until the final composition is obtained.

9. Dosage regimen for the administration of water-soluble therapeutically active ingredients in the form of a tablet and/or immediate release capsule for oral use to patients with difficulty swallowing or dysphagia which provides the administration to said patients of a composition comprising a semisolid preparation comprising hydroxypropyl methylcellulose in a percentage ranging from 1 to 6% by weight with respect to the total composition, a pharmacologically effective amount of at least one water-soluble therapeutically active ingredient and suitable pharmaceutically acceptable excipients, wherein the water-soluble therapeutically active ingredient is in the form of tablet and/or immediate release capsule for oral use, dispersed in the semisolid preparation.
